# EUROPEAN PATENT APPLICATION

(11) **EP 3 398 586 A2**
(43) Date of publication of application: **07.11.2018**
(21) Application number: 16882143.7
(22) Date of filing: 30.12.2016
(51) Int. Cl.: A61K 9/08, A61K 47/10, A61K 47/12, A61K 47/18, A61K 47/26, A61K 47/34, A61K 38/27, C07K 14/61

(54) **NOVEL LIQUID-PHASE PREPARATION OF LONG-ACTING HUMAN GROWTH HORMONE CONJUGATE**

(30) Priority: 30.12.2015 KR 20150189919
(71) Applicant: Hanmi Pharm. Co., Ltd., Hwaseong-si, Gyeonggi-do 18536 (KR)
(72) Inventor: LIM, Hyung Kyu, Hwaseong-si Gyeonggi-do 18469 (KR); DONG, Joo Young, Hwaseong-si Gyeonggi-do 18469 (KR); BAE, Sung Min, Hwaseong-si Gyeonggi-do 18469 (KR); KWON, Se Chang, Hwaseong-si Gyeonggi-do 18469 (KR)
(74) Representative: Andrews, Robert
(86) International application number: PCT/KR2016/015534
(87) International publication number: WO 2017/116191

(57) **Abstract**

The present invention relates to a liquid formulation of a long-acting human growth hormone conjugate and a preparation method thereof.

## Description

### [Technical Field]

The present invention relates to a liquid formulation of a long-acting human growth hormone conjugate and a preparation method thereof.

### [Background Art]

Human growth hormone (hereinafter referred to as "hGH") is a polypeptide hormone with a molecular weight of about 22,000, consisting of 191 amino acids, produced in human pituitary gland, and is mainly used for treating pituitary dwarfism. Conventionally, the hGH extracted from the human pituitary gland has been used, but only a very limited number of people have been treated due to its limited supply. Additionally, use of the hGH extracted from the pituitary gland has been banned since it was reported that a fatal degenerative neurological disorder, Creutzfeldt-Jacob disease, was found in some of patients treated with the hGH extracted from the pituitary gland. Recently, development of genetic engineering techniques has enabled hGH production in *E. coli* and yeast, and recombinant hGH formulations produced therefrom have been approved in several countries since 1985 and have become commercially available after passing toxicological and clinical tests.

A polypeptide such as the hGH has a low stability and thus is easily denatured, and is degraded by serum protease in blood and thus is easily removed via kidney or liver. Consequently, a protein drug containing the polypeptide as a pharmaceutical ingredient must be frequently administered to a patient to maintain its blood concentration and titer. In a case where the protein drug is administered in a form of an injection to a patient, however, frequent injections of the protein drugs to maintain ablood concentration of the active polypeptides cause much pain to the patient. To solve such problems, there have been many attempts to increase stability of the protein drug in blood and maintain its blood concentration at a high level for a long period of time to maximize drug efficacy. It is preferable that a long-acting formulation of such protein drug increases the stability of the protein drug, maintains a high titer of the drug, and does not cause an immune reaction.

Meanwhile, Korean Patent Nos. 10-0567902 and 10-0725315 disclosed a conjugate prepared by linking an immunoglobulin Fc region and a non-peptidyl polymer with a physiologically active polypeptide as a long-acting formulation of a protein drug capable of minimizing a decrease in protein activity and increasing protein stability.

hGH may be used as a physiologically active polypeptide using the method above to prepare a long-acting hGH conjugate. It is essential to prevent a physicochemical change such as denaturation, aggregation, adsorption, or hydrolysis due to a degradation induced by light, heat, or impurities in additives during storage and transport processes, while maintaining *in vivo* activity of the hGH. Since the long-acting hGH conjugate has a larger size and increased molecular weight compared to the hGH polypeptide, it is difficult to stabilize the conjugate compared to the hGH polypeptide.

Being unstable in a liquid formulation, such stability problem of the protein has been solved by lyophilization. The lyophilized protein formulation has an advantage of maintaining long-term stability, but is inconvenient in that it must be reconstituted with a solubilizing agent for an injection and has problems in that production of polymers during the reconstitution or lyophilization causes a loss of activity, and that it takes much time and cost to lyophilize protein.

In order to develop a stable liquid hGH formulation, it is important to control a degradation pattern of hGH, such as a rate of degradation product production by deamidation, polymer production, and oxidation.

Although temperature, pH, and additives of the liquid formulation affect the production rate of the degradation products, a composition capable of stabilizing all proteins to be clinically applied is still unknown. The liquid formulation stabilizing a particular protein may not be applied to stabilization of other proteins. For maximizing stability of a protein in liquid, specifically hGH, it is well known in the art that this must be extensively preceded by selecting a factor or additive capable of minimizing a production rate of a specific breakdown product of the protein or a combination thereof.

Additionally, different proteins, due to physicochemical differences thereof, may be inactivated gradually under different ratios and conditions during storage. That is, effects on prolongation of shelf-life due to a substance used for stabilization are not equivalent for different proteins, and as a result, the stabilizer used for providing shelf-life stability has various ratios, concentrations, and types depending on physicochemical properties of a target protein. Also, when used in combination, the stabilizer may cause adverse effects due to competitive interactions therebetween and side effects, rather than intended effects. Furthermore, properties and concentration of the stored protein may change during its storage, causing different effects. The protein stabilization in solution, therefore, requires many efforts and precautions.

In particular, since the long-acting hGH conjugate with an improved *in vivo* durability and stability is in a form in which the hGH, a physiologically active peptide, is linked to an immunoglobulin Fc region, its molecular weight and volume are greatly different from those of the general hGH, thereby requiring a special composition for the protein stabilization. Additionally, since the physiologically active peptide hGH and the immunoglobulin Fc region are peptides or proteins with different physicochemical properties, they should be stabilized simultaneously. As described above, however, different peptides or proteins may be gradually inactivated under different ratios and conditions due to difference in their physicochemical properties during storage. Also, when stabilizers suitable for each peptide or protein are used in combination, they may cause adverse effects due to the competitive interactions therebetween and the side effects, rather than the intended effects. Therefore, for a long-acting hGH conjugate, it is difficult to find a composition of the stabilizer capable of stabilizing both the physiologically active peptide hGH and the immunoglobulin Fc region simultaneously.

### [Disclosure]

### [Technical Problem]

The present inventors have made extensive efforts to find a composition for an improved stability and decreased precipitation of the protein for a prolonged storage of the long-acting hGH conjugate without contamination of a pathogenic virus. As a result, it has been confirmed that using a stabilizer comprising a buffer, sugar alcohol, and non-ionic surfactant, without an isotonic agent, may increase a stability of a long-acting hGH conjugate, thereby producing an economical and stable liquid formulation. Furthermore, the present inventors have confirmed that when the liquid formulation further comprises a preservative, a liquid formulation for multiple administration capable of maintaining a high stability of the long-acting hGH conjugate may be provided, thereby completing the present invention.

### [Technical Solution]

An object of the present invention is to provide a liquid formulation of a long-acting hGH conjugate, comprising a long-acting hGH conjugate and an albumin-free stabilizer comprising a buffer solution, sugar alcohol, non-ionic surfactant, and preservative.

Another object of the present invention is to provide a method for preparing the liquid formulation of the long-acting hGH conjugate.

Still another object of the present invention is to provide a use of the liquid formulation of the long-acting hGH conjugate for preparation a medicament for treatment of pituitary dwarfism, growth hormone deficiency, Prader-Willi syndrome, or idiopathic short stature.

Still another object of the present invention is to provide a method for treating pituitary dwarfism, growth hormone deficiency, Prader-Willi syndrome, or idiopathic short stature, comprising administering the liquid formulation of the long-acting hGH conjugate to a subject in need thereof.

### [Advantageous Effects]

Since the liquid formulation of the long-acting hGH conjugate according to the present invention does not comprise human serum albumin and any potentially hazardous factor, there is no concern of viral contamination. Additionally, the long-acting hGH conjugate formed by linking hGH to an immunoglobulin Fc region has a larger molecular weight and improved *in vivo* physiological durability compared to native provides, and the liquid formulation of the conjugate provides an excellent stability for the long-acting hGH conjugate. The liquid formulation may be a stable liquid formulation comprising a preservative capable of multiple administrations. Such liquid formulation of the present invention, as a simple formulation, shows an excellent shelf-life stability and thus may be provided more economically than other stabilizers or lyophilizing formulation. It may be also used as an efficient drug formulation as *in vivo* protein activity is maintained for a long time compared to a conventional hGH formulation.

### [Description of Drawings]

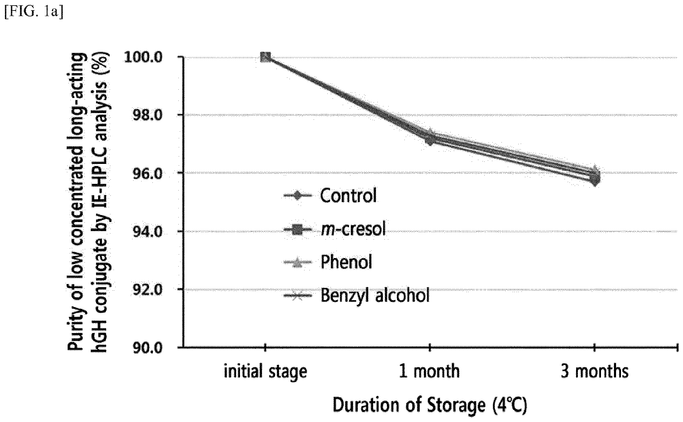

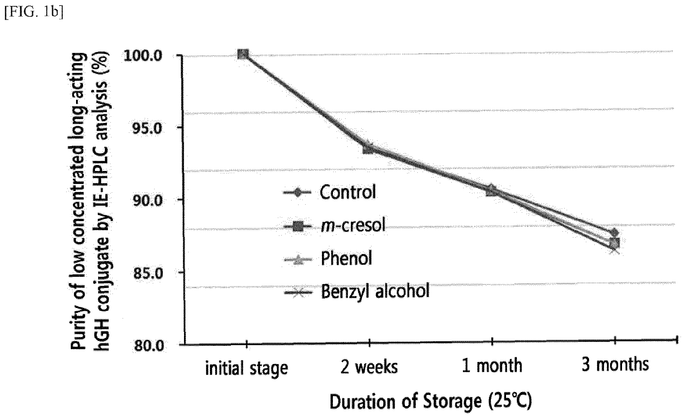

FIGS. 1a and 1b are results of experiments on effects of the preservative-containing liquid formulation for multiple administration according to the present invention on the stability of a low-concentration long-acting hGH conjugate. FIGS. 1a and 1b are graphs analyzed by IE-HPLC after storage at 4°C and 25°C, respectively. IE-HPLC (%) refers to (Area %/Start Area %), a residual rate of the low-concentration long-acting hGH conjugate relative to an initial value. The results show that there is no significant difference in stability between when m-cresol, phenol, or benzyl alcohol is included as a preservative and when there is no preservative included.

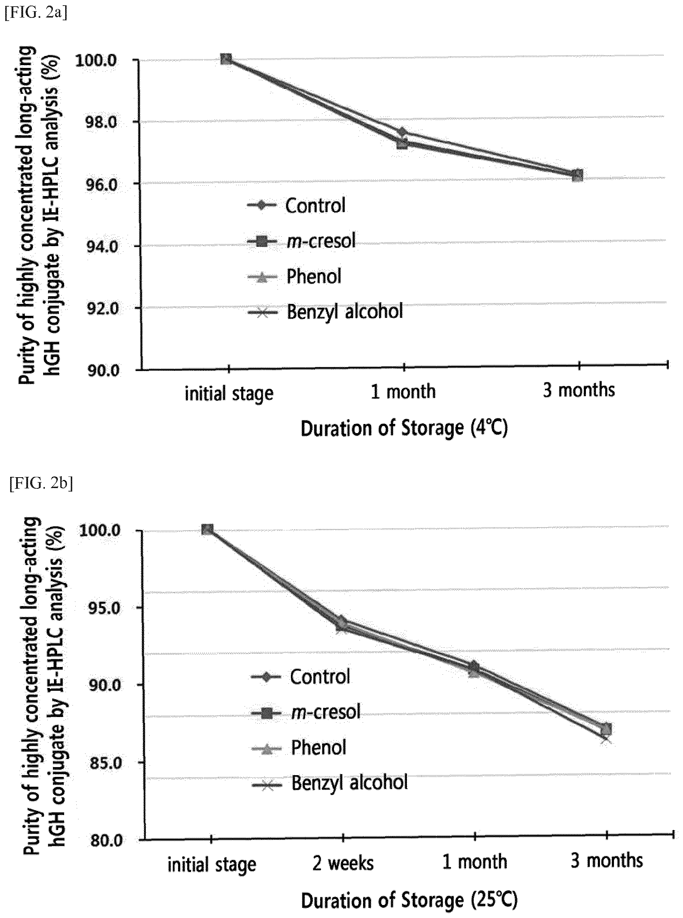

FIGS. 2a and 2b are results of experiments on effects of the preservative further included for multiple administrations, based on the liquid formulation of FIGS. 1, on the stability of a high-concentration long-acting hGH conjugate. FIGS. 2a and 2b are graphs analyzed by IE-HPLC after storage at 4°C and 25°C, respectively. IE-HPLC (%) refers to (Area %/Start Area %), a residual rate of the high-concentration long-acting hGH conjugate relative to an initial value. Like the results of FIGS. 1a and 1b, the results show that there is no significant difference in stability between when m-cresol, phenol, and benzyl alcohol are included as a preservative and when there is no preservative included.

### [Best Mode]

To achieve the above objects, an exemplary embodiment of the present invention is a liquid formulation of a long-acting hGH conjugate comprising (i) a long-acting human growth hormone(hGH) conjugate, and (ii) an albumin-free stabilizer comprising a buffer solution, sugar alcohol, non-ionic surfactant, and preservative.

As a specific example, the albumin-free stabilizer does not comprise an isotonic agent, e.g. sodium chloride.

As another specific example, the long-acting hGH conjugate is a conjugate in which hGH is linked to an immunoglobulin Fc region.

As another specific example, the long-acting hGH conjugate is in a form of a fusion protein in which the hGH and immunoglobulin Fc region are linked via a non-peptidyl polymer or using a recombination technique.

As another specific example, the non-peptidyl polymer is selected from the group consisting of polypropylene glycol, polyethylene glycol, a copolymer of ethylene glycol and propylene glycol, polyoxyethylated polyol, polyvinyl alcohol, polysaccharide, dextran, polyvinyl ethyl ether, a biodegradable polymer such as polylactic acid (PLA) and polylactic-glycolic acid (PLGA), a lipid polymer, chitins, hyaluronic acid, and a combination thereof.

As another specific example, the non-peptidyl polymer is polyethylene glycol.

As another specific example, the buffer is a citrate buffer or acetate buffer.

As another specific example, the buffer has a pH in the range of 4.0 to 7.0.

As another specific example, the sugar alcohol is at least one selected from the group consisting of mannitol, sucrose, and sorbitol.

As another specific example, the sugar alcohol has a concentration in a range of 1% (w/v) to 10% (w/v) relative to the total volume of the liquid formulation.

As another specific example, the non-ionic surfactant is polysorbate or poloxamer.

As another specific example, the non-ionic surfactant has a concentration in a range of 0.001 % (w/v) to 0.1% (w/v) relative to the total volume of the liquid formulation.

As another specific example, the preservative is at least one selected from the group consisting of m-cresol, phenol, and benzyl alcohol.

As another specific example, the preservative has a concentration in a range of 0.01% to 1.0% (w/v) relative to the total volume of the liquid formulation.

As another specific example, the liquid formulation is for multiple administration.

As another specific example, the long-acting hGH conjugate has a concentration in a range of 1 mg/mL to 100 mg/mL.

As another specific example, the stabilizer further comprises at least one selected from the group consisting of saccharides, polyhydric alcohol, and amino acids.

As another specific example, the hGH is hGH having the same amino acid sequence as that of native hGH, hGH having a modification on the amino acid sequence of the native hGH, or a derivative having a similar activity to that of native hGH.

As another specific example, the liquid formulation of the long-acting hGH conjugate comprises (i) a long-acting hGH conjugate in which the hGH is linked to an immunoglobulin Fc region, and (ii) an albumin-free stabilizer consisting of a buffer solution, sugar alcohol, non-ionic surfactant, and preservative.

As another specific example, the buffer is a citrate buffer, the sugar alcohol is mannitol, the surfactant is polysorbate 80, and the preservative is selected from the group consisting of m-cresol, phenol, and benzyl alcohol.

As another specific example, the immunoglobulin Fc region is derived from IgG, IgA, IgD, IgE, or IgM.

As another specific example, each domain of the immunoglobulin Fc region is a hybrid of domains with different origins derived from an immunoglobulin selected from the group consisting of IgG, IgA, IgD, IgE, and IgM.

As another specific example, the immunoglobulin Fc region is a dimer or multimer composed of single-chain immunoglobulins consisting of domains with the same origin.

As another specific example, the immunoglobulin Fc region is an aglycosylated human IgG4 Fc region.

As another specific example, the formulation is for treating pituitary dwarfism, growth hormone deficiency, Prader-Willi syndrome, or idiopathic short stature.

Another exemplary embodiment of the present invention is a method for preparing the liquid formulation of the long-acting hGH conjugate, comprising mixing the long-acting hGH conjugate in which the hGH is linked to the immunoglobulin Fc region with a buffer, sugar alcohol, non-ionic surfactant, and preservative.

Still another exemplary embodiment of the present invention is a use of the liquid formulation of the long-acting hGH conjugate for preparation a medicament for treatment of pituitary dwarfism, growth hormone deficiency, Prader-Willi syndrome, or idiopathic short stature.

Still another exemplary embodiment of the present invention is a method for treating pituitary dwarfism, growth hormone deficiency, Prader-Willi syndrome, or idiopathic short stature, comprising administering the liquid formulation of the long-acting hGH conjugate to a subject in need thereof.

### [Mode for Invention]

An exemplary embodiment of the present invention is to provide a liquid formulation of a long-acting human growth hormone(hGH) conjugate comprising (i) an hGH conjugate in which the hGH is linked to an immunoglobulin Fc region, and (ii) an albumin-free stabilizer comprising a buffer solution, sugar alcohol, non-ionic surfactant, and preservative.

Hereinafter, the long-acting hGH conjugate and constitutional ingredients thereof are described in detail. Further, each description and embodiment disclosed in the present invention may be applied herein to describe different descriptions and embodiments. In other words, all combinations of various components disclosed in the present invention are included within the scope of the present invention.

As used herein, the term "long-acting human growth hormone (hGH) conjugate" refers to a conjugate, as a protein in which hGH, a physiologically active peptide, is linked to a carrier, with an improved *in vivo* physiological duration compared to native hGH. Specifically, the conjugate may be a protein in which the physiologically active peptide hGH is linked to an immunoglobulin Fc region.

The carrier may be selected from the group consisting of an immunoglobulin Fc region, albumin, transferrin, and PEG, and specifically is an immunoglobulin Fc region, but is not limited thereto.

As used herein, the term "long-acting" refers to an improved physiological activity durability compared to native hGH, whereas the term "conjugate" refers to a form in which hGH is linked to a carrier.

Specifically, the long-acting hGH conjugate is in a form in which hGH and a carrier, which is specifically an immunoglobulin Fc region, are linked via a non-peptidyl polymer or a form of a fusion protein in which hGH and a carrier in a form of a protein, which is specifically an immunoglobulin Fc region, are linked using a recombination technique, but is not limited thereto. In the case of the fusion protein, a form in which hGH and a carrier in a form of protein, specifically an immunoglobulin Fc region, are linked via a peptide linker is included in the scope of the present invention.

In the present invention, the hGH includes hGH having the same amino acid sequence as that of the native hGH, hGH having a modification on the amino acid sequence of the native hGH, or a peptide derivative having a similar activity to that of the native hGH. Examples of the modification include substitution, deletion, and insertion of an amino acid constituting hGH, but are not limited thereto, and the amino acid sequence may be modified as long as its biological activity is not significantly affected.

Additionally, the hGH may be a native or recombinant form, specifically recombinant hGH prepared using microorganisms as host cells, but is not limited thereto. An example of the microorganism is *E. coli.*

The hGH sequence may be obtained from common database such as NCBI GenBank. Additionally, the hGH may have an amino acid sequence homology of 70% or higher, specifically 80% or higher, more specifically 90% or higher, even more specifically 95% or higher, and most specifically 98% or higher to an amino acid sequence of native hGH, as long as it has an hGH activity.

As used herein, the term "non-peptidyl polymer" refers to a biocompatible polymer in which one or more repeating units are linked, and the repeating units are connected to each other by any covalent bond other than peptide bond. In the present invention, the term non-peptidyl polymer may be used interchangeably with the term non-peptidyl linker.

The non-peptidyl polymer may be selected from the group consisting of a biodegradable polymer including polyethylene glycol, polypropylene glycol, an ethylene glycol-propylene glycol copolymer, polyoxyethylated polyol, polyvinyl alcohol, polysaccharide, dextran, polyvinyl ethyl ether, polylactic acid (PLA), and polylactic-glycolic acid (PLGA), a lipid polymer, chitin, hyaluronic acid, and a combination thereof. Specifically, the non-peptidyl polymer is polyethylene glycol, but is not limited thereto. Additionally, derivatives which are already known in the art and those which can be easily prepared within the skill in the art are included in the non-peptidyl polymer in the scope of the present invention.

The peptide linker which is used in the fusion protein obtained by a conventional inframe fusion method has a drawback in that it is easily cleaved *in vivo* by a proteolytic enzyme, and thus an effect of increasing the blood half-life of the active drug by a carrier cannot be obtained as expected. However, when a polymer having resistance to the proteolytic enzyme is used, the blood half-life of the peptide similar to that of the carrier may be maintained. Therefore, any non-peptidyl polymer may be used without limitation as long as it is a polymer having the above-mentioned function, that is, a polymer having resistance to the *in vivo* proteolytic enzyme. The non-peptidyl polymer has a molecular weight ranging from 1 kDa to 100 kDa, and specifically from 1 kDa to 20 kDa, but is not limited thereto. Additionally, the non-peptidyl polymer of the present invention linked to the immunoglobulin Fc region may be one type of polymer or a combination of different types of polymers.

As used herein, the term "immunoglobulin Fc region" refers to a heavy-chain constant region 2 (C_{H}2) and the heavy-chain constant region 3 (C_{H}3), excluding variable regions of the heavy and light chains, heavy-chain constant region 1 (C_{H}1), and light-chain constant region 1 (C_{L}1) of the immunoglobulin. Also, the immunoglobulin Fc region of the present invention may be an extended Fc region that comprises a portion or the entirety of the heavy-chain constant region 1 (C_{H}1) and/or the light-chain constant region 1 (C_{L}1) except the variable regions of the heavy and light chains of the immunoglobulin, as long as it has substantially the equivalent or an improved effect compared to the native Fc region. Additionally, the immunoglobulin Fc region may be a fragment wherein a considerably long portion of the amino acid sequence corresponding to C_{H}2 and/or C_{H}3 is deleted. That is, the immunoglobulin Fc region of the present invention may comprise 1) a C_{H}1 domain, a C_{H}2 domain, a C_{H}3 domain, and a C_{H}4 domain, 2) a C_{H}1 domain and a C_{H}2 domain, 3) a C_{H}1 domain and a C_{H}3 domain, 4) a C_{H}2 domain and a C_{H}3 domain, 5) a combination of at least one domain and an immunoglobulin hinge region (or a portion of the hinge region), or 6) a dimer of each domain of the heavy-chain constant region and light-chain constant region.

As the immunoglobulin Fc region has a relatively low molecular weight compared to the entire immunoglobulin molecule, it is preferable in terms of preparation, purification, and production yield of a conjugate. Further, since the Fab regions which show a high heterogeneity due to a different amino acid sequence for each antibody are removed, it can be expected that homogeneity significantly increases and possibility of antigenicity in blood decreases.

The immunoglobulin Fc region of the present invention comprises not only a native amino acid sequence and but also a derivative (mutant) thereof. The amino acid sequence derivative refers to having a different sequence from the native sequence by deletion, insertion, non-conservative or conservative substitution of at least one amino acid residues of the native amino acid sequence, or a combination thereof. For example, in IgG Fc, amino acid residues at positions 214 to 238, 297 to 299, 318 to 322, or 327 to 331, which are known to be important for protein binding, may be a suitable target for modification.

Additionally, other types of derivatives may be used, including the derivatives in which a region capable of forming a disulfide bond is removed, a few amino acid residues at the N-terminal of the native Fc are removed, or a methionine residue is added at the N-terminal of the native Fc. Further, in order to eliminate an effector function, a complement-binding site, *e.g.,* a clq-binding site or antibody-dependent cell-mediated cytotoxicity (ADCC) site, may be removed. Techniques for preparing such a sequence derivative of the immunoglobulin Fc region are disclosed in WO 97/34631 and WO 96/32478.

Exchanges of amino acids in proteins and peptides which do not modify overall molecular activities are known in the art (H. Neurath, R. L. Hill, The Proteins, Academic Press, New York, 1979). The most commonly occurring exchanges are Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly, in both directions. In some cases, the Fc region may be modified by phosphorylation, sulfation, acrylation, glycosylation, methylation, farnesylation, acetylation, and amidation.

The Fc derivatives demonstrate the same biological activity as the Fc region of the present invention, and they have an enhanced structural stability against heat and pH.

Additionally, these Fc regions may be obtained from the native proteins isolated from humans or other animals such as cows, goats, swine, mice, rabbits, hamsters, rats, and guinea pigs, or may be recombinants obtained from transformed animal cells or microorganisms, or derivatives thereof. A method of obtaining Fc regions from the native forms may include isolating an entire immunoglobulin from a human or animal and treating it with protease. The immunoglobulins are cleaved into Fab and Fc regions when treated with papain, and into pF'c and F(ab)₂ when treated with pepsin. Fc or pF'cmay be separated by size exclusion chromatography to isolate Fc or pF'c. Specifically, the Fc region may be a recombinant immunoglobulin Fc region in which the human-derived immunoglobulin Fc region is obtained from a microorganism, but is not limited thereto.

In addition, the immunoglobulin Fc region may be in the form of having native sugar chains, increased sugar chains compared to a native form, or decreased sugar chains compared to the native form, or may be in a deglycosylated form. The increase, decrease or removal of the immunoglobulin Fc sugar chains may be performed by using common methods in the art including chemical methods, enzymatic methods, and genetic engineering method using a microorganism. Herein, an immunoglobulin Fc region in which a sugar chain is removed from the Fc has a drastically decreased binding affinity to clq and as well as decreased or eliminated antibody-dependent cytotoxicity or complement-dependent cytotoxicity, and thus unnecessary immune responses *in vivo* may be avoided. In this regard, an immunoglobulin Fc region in a deglycosylated or aglycosylated form may be more suitable to the object of the present invention as a drug carrier.

As used herein, the term "deglycosylation" refers to an Fc region with sugar removed by an enzyme, whereas the term "aglycosylation" refers to an Fc region which is produced in a prokaryote, specifically *E. coli,* and thus is not glycosylated.

Meanwhile, the immunoglobulin Fc region may originate from humans or other animals including cows, goats, swine, mice, rabbits, hamsters, rats, and guinea pigs, specifically from humans, but is not limited thereto.

Additionally, the immunoglobulin Fc region may be an Fc region derived from IgG, IgA, IgD, IgE, or IgM, or prepared by a combination or hybrid thereof. Specifically, it may be derived from IgG or IgM, which is the most abundant in human blood, and more specifically from IgG, which is known to improve half-life of a ligand-binding protein.

As used herein, the term "combination" refers to a linkage between a polypeptide encoding single-chain immunoglobulin Fc regions of the same origin and a single-chain polypeptide of different origin when forming a dimer or multimer. That is, a dimer or multimer can be formed from two or more fragments selected from the group consisting of IgG Fc, IgA Fc, IgM Fc, IgD Fc, and IgE Fc fragments.

As used herein, the term "hybrid" refers to at least two sequences corresponding to immunoglobulin Fc fragments of different origins present in a single-chain immunoglobulin Fc region. In the present invention, various types of the hybrids may be used. In other words, a hybrid of domains may be composed of one to four domains selected from the group consisting of C_{H}1, C_{H}2, C_{H}3, and C_{H}4 of IgG Fc, IgM Fc, IgA Fc, IgE Fc, and IgD Fc, and may comprise a hinge region.

Meanwhile, IgG may also be divided into subclasses IgG1, IgG2, IgG3, and IgG4, and a combination or hybrid thereof is also possible in the present invention. Specifically, IgG may be the subclasses IgG2 and IgG4, and more specifically an Fc region of IgG4, which barely has an effector function, such as complement dependent cytotoxicity (CDC).

Still more specifically, an immunoglobulin Fc region as a drug carrier of the present invention is a human IgG4-derived non-glycosylated Fc region. The human-derived Fc region is preferred to a non-human-derived Fc region, which can act as an antigen in the human body and cause undesirable immune responses such as production of new antibodies against the antigen.

The long-acting hGH conjugate used in the present invention may be prepared by linking hGH prepared from a native or recombinant form by any method to an immunoglobulin Fc region prepared by treating native IgG with a certain protease or produced from a transformed cell using the recombination technique. As a linking method used for this purpose, the conjugate may be prepared by cross-linking the hGH and the immunoglobulin Fc region using a non-peptidyl polymer or may be produced as a fusion protein in which the hGH and the immunoglobulin Fc region are linked using the recombination technique.

The specifications of Korean Patent Registration No. 10-0725315 (protein conjugate using an immunoglobulin fragment and method for the preparation thereof) and US Patent Application Publication No. 2013-0288333 are included as Cited References of the present invention for the preparation of the long-acting hGH conjugate used in the present invention.

Hereinafter, the composition of the liquid formulation of the long-acting hGH conjugate will be described in detail. As described above, each description and embodiment disclosed in the present invention can be applied to every other description and embodiment. In other words, all combinations of various elements disclosed in the present invention are included within the scope of the present invention.

As used herein, the term "liquid formulation of a long-acting hGH conjugate" refers to a liquid formulation which comprises the long-acting hGH conjugate. The liquid formulations include liquid formulations for both internal and external application. In the present invention, the liquid formulation of the long-acting hGH conjugate may comprise a pharmaceutically effective amount of the long-acting hGH conjugate. In general, the pharmaceutically effective amount of the hGH is about 1 mg to 3 mg in a single-use vial, but is not limited thereto.

A concentration of the long-acting hGH conjugate contained in the liquid formulation of the present invention may be 1 mg/mL to 200 mg/mL, specifically 1 mg/mL to 100 mg/mL, more specifically 10 mg/mL to 58.5 mg/mL, but is not limited thereto. The liquid formulation of the long-acting hGH conjugate of the present invention may store the long-acting hGH conjugate stably without precipitation when the long-acting hGH conjugate is comprised at a low concentration as well as a high concentration, enabling supply of the high-concentration hGH present *in vivo.*

The liquid formulation of the long-acting hGH conjugate of the present invention may comprise the long-acting hGH conjugate in a pharmacologically effective amount and an albumin-free stabilizer.

As used herein, the term "stabilizer" refers to an agent comprising an ingredient which allows the long-acting hGH conjugate to be stored stably. With regard to proteins such as the long-acting hGH conjugate, the shelf-life stability is important for ensuring dose accuracy and suppressing a potential formation of antigenic substances such as aggregates of the long-acting hGH conjugate.

It is preferable that the stabilizer comprises a buffer, sugar alcohol, and non-ionic surfactant, and it may further comprise a preservative. More specifically, the stabilizer may essentially consist of a buffer, sugar alcohol, non-ionic surfactant, and preservative, but is not limited thereto. Additionally, the stabilizer may not comprise an isotonic agent such as sodium chloride, but is not limited thereto.

The buffer plays a role in maintaining a pH of the liquid formulation to prevent a drastic fluctuation of the pH of the liquid formulation for the stability of the long-acting hGH conjugate.

The buffer may include an alkaline salt (e.g., sodium or potassium phosphate, or monobasic or dibasic salts thereof), sodium citrate/citric acid, sodium acetate/acetic acid, and any other pharmaceutically acceptable pH buffering agent known in the art, as well as a combination thereof.

For the purpose of the present invention, the buffer is more specifically an acetic acid or citric acid buffer (e.g., sodium acetate or sodium citrate), and still more specifically a citrate buffer.

The acetate or citrate composing the acetate or citrate buffer has a concentration in a rage of 1 mM to 100 mM, more specifically 3 mM to 100 mM, but is not limited thereto.

The buffer has a pH in a range of 4.0 to 8.0, more specifically 4.0 to 7.0, but is not limited thereto.

The sugar alcohol may play a role in improving the stability of the long-acting hGH conjugate.

The sugar alcohol used in the present invention has a concentration specifically in a range of 1% (w/v) to 20% (w/v), and more specifically 1% (w/v) to 10% (w/v), relative to the total volume of the liquid formulation.

The sugar alcohol may be at least one selected from the group consisting of mannitol, sucrose, and sorbitol. The sugar alcohol may specifically be mannitol or sorbitol, and more specifically mannitol, but is not limited thereto.

The non-ionic surfactant plays a role in preventing protein from being adsorbed onto a hydrophobic surface or from aggregating by lowering surface tension of the protein solution.

Specific examples of the non-ionic surfactant which may be used in the present invention are a polysorbate-type non-ionic surfactant, poloxamer-type non-ionic surfactant, and a combination of one or two thereof. Specifically, polysorbate-type non-ionic surfactant may be used, but the non-ionic surfactant is not limited thereto.

Examples of the polysorbate-type non-ionic surfactant are polysorbate 20, polysorbate 40, polysorbate 60, and polysorbate 80, but are not limited thereto. Specifically, polysorbate 80 may be used, but the non-ionic surfactant is not limited thereto.

The liquid formulation of the present invention may comprise the non-ionic surfactant at a low concentration below 0.1% (w/v), and more specifically in a range of 0.001% (w/v) to 0.1 % (w/v), but is not limited thereto.

The stabilizer of the present invention preferably does not comprise albumin. As human serum albumin, which may be used as a stabilizer of protein, is produced from human blood, there is a risk of contamination by human-derived pathogenic viruses. There is a possibility that gelatin or bovine serum albumin may cause a disease or induce an allergic response in some patients. In contrast, since the albumin-free stabilizer of the present invention does not comprise heterologous protein such as human- or animal-derived serum albumin or purified gelatin, there is no risk of viral infection.

Additionally, the stabilizer of the present invention may further comprise saccharides, polyhydric alcohols, and neutral amino acids.

In order to increase the shelf-life stability of the long-acting hGH conjugate, saccharides and polyhydric alcohols may be further included. Specific examples of the saccharides include monosaccharides such as mannose, glucose, fucose, and xylose and polysaccharides such as lactose, maltose, sucrose, raffinose, and dextran. Those of the polyhydric alcohols include propylene glycol, low-molecular-weight polyethylene glycol, glycerol, low-molecular-weight polypropylene glycol, and a combination thereof.

The liquid formulation of the long-acting hGH conjugate of the present invention may further comprise a preservative to prevent microbial contamination of a formulation for multiple administration in addition to the conjugate, buffer, sugar alcohol, and non-ionic surfactant described above.

As used herein, the term "preservative" refers to a compound added in a pharmaceutical formulation to function as an antibacterial agent. Examples of the preservative include benzethonium, chlorohexidine, phenol, m-cresol, benzyl alcohol, methylparaben, propylparaben, chlorobutanol, o-cresol, p-cresol, chlorocresol, benzalkonium chloride, phenylmercuric nitrate, thimerosal, and benzoic acid, but are not limited thereto.

The preservatives may be used individually or in a random combination of two or more. Specifically, the liquid formulation of the present invention may comprise at least one selected from m-cresol, phenol, and benzyl alcohol as a preservative. For the liquid formulation of the present invention, the preservative may be included in an amount of 0.01% (w/v) to 3% (w/v), specifically 0.1% (w/v) to 1.0% (w/v), but is not limited thereto.

In a specific example of the present invention, each of phenol, m-cresol, and benzyl alcohol was included in the liquid formulation of the present invention in an amount of 0.1% (w/v) to 1.0% (w/v) and its effect on the stability of the long-acting hGH conjugate was analyzed. As a result, the stability of the conjugate was maintained without precipitation of the long-acting hGH conjugate.

Accordingly, the novel liquid formulation of the long-acting hGH conjugate in which the stabilizer further comprises the preservative described above may be used for multiple administration.

In addition to the buffer, sugar alcohol, non-ionic surfactant, and preservative described above, other ingredients or substances known in the art may be selectively included in the liquid formulation of the present invention as long as the effect of the present invention is not affectd.

More specifically, the liquid formulation may comprise (i) a long-acting hGH conjugate in which the hGH is linked to an immunoglobulin Fc region; and (ii) an albumin-free stabilizer consisting of a buffer solution, sugar alcohol, non-ionic surfactant, and preservative, but is not limited thereto.

Herein, the buffer may be a citrate buffer, the sugar alcohol may be mannitol, the surfactant maybe polysorbate 80, and the preservative may be selected from the group consisting of m-cresol, phenol, and benzyl alcohol.

Still more specifically, the liquid formulation may comprise 20 mM of sodium citrate at pH 5.2, 4% mannitol, 0.005% polysorbate 80, and m-cresol, phenol, or benzyl alcohol as a preservative when the concentration of the long-acting hGH conjugate is between 10.0 mg/mL and 58.5 mg/mL.

Additionally, the formulation may be for treating pituitary dwarfism, growth hormone deficiency, Prader-Willi syndrome, or idiopathic short stature. That is, the formulation may be a pharmaceutical composition.

In another aspect, the present invention provides a method for preparing the liquid formulation.

The liquid formulation and its ingredients are as described above.

The preparation method may comprise mixing a long-acting hGH conjugate in which hGH is linked to an immunoglobulin Fc region with a buffer, sugar alcohol, non-ionic surfactant, and preservative.

More specifically, the preparation method of the liquid formulation of the present invention may comprise formulating a long-acting hGH conjugate and mixing the formulated long-acting hGH conjugate with a stabilizer comprising a buffer, sugar alcohol, and non-ionic surfactant. Additionally, a stable liquid formulation of a long-acting hGH conjugate for multiple administration may be prepared by adding a preservative to the stabilizer.

According to an embodiment of the present invention, the stabilizer comprising sodium citrate as a buffer, mannitol as a sugar alcohol, polysorbate 80 as a surfactant, and m-cresol, phenol, or benzyl alcohol as a preservative was shown to be a stabilizer which provides stability for both low- and high-concentration long-acting hGH conjugates without precipitation. That is, a stable liquid formulation may be prepared without precipitation when the concentration of the long-acting hGH conjugate is between 10.0 mg/mL and 58.5 mg/mL.

In still another aspect, the present invention provides a use of the liquid formulation of the long-acting hGH conjugate for preparation a medicament for treatment of pituitary dwarfism, growth hormone deficiency, Prader-Willi syndrome, or idiopathic short stature, comprising (a) a long-acting hGH conjugate in which the hGH is linked to an immunoglobulin Fc region, and (b) an albumin-free stabilizer comprising a buffer solution, sugar alcohol, non-ionic surfactant, and preservative,

The liquid formulation and its ingredients are as described above.

The liquid formulation of the long-acting hGH conjugate comprising hGH may have effects of ameliorating, preventing, or treating pituitary dwarfism, growth hormone deficiency, Prader-Willi syndrome, or idiopathic short stature.

In still another aspect, the present invention provides a method for treating pituitary dwarfism, growth hormone deficiency, Prader-Willi syndrome, or idiopathic short stature, comprising administering the liquid formulation of the long-acting hGH conjugate to a subject in need thereof, comprising (a) a long-acting hGH conjugate in which the hGH is linked to an immunoglobulin Fc region, and (b) an albumin-free stabilizer comprising a buffer solution, sugar alcohol, non-ionic surfactant, and preservative.

The liquid formulation and its ingredients are as described above.

Hereinbelow, the present invention will be described in detail with reference to Examples below. The Examples are disclosed only for illustrative purposes and should not be construed as limiting the scope of the present invention.

### Preparation Example: Preparation of the long-acting hGH conjugate

ALD-PEG-ALD, a polyethylene glycol with a molecular weight of about 3.4 kDa having aldehyde functional groups at both ends, was conjugated with hGH (molecular weight 22 kDa), and then linked to the N-terminal of a human IgG4-derived aglycosylated Fc region (about 50 kDa) to prepare an hGH-PEG-Fc conjugate (hereinafter, referred to as "long-acting hGH conjugate"), which is a representative long-acting hGH conjugate of the present invention. The long-acting hGH conjugate was then purified.

### Example 1: Evaluation of stability of liquid formulation of low-concentration long-acting hGH conjugate according to preservatives

The present inventors have invented a liquid formulation of a long-acting hGH conjugate comprising a pharmacologically effective amount of the long-acting hGH conjugate in which a physiologically active peptide hGH is linked to an and immunoglobulin Fc region, a buffer, sugar alcohol, surfactant, and isotonic agent (Korean Application Publication No. 10-2010-0067796), and a liquid formulations of high- and low-concentration long-acting hGH conjugates comprising a pharmacologically effective amount of long-acting hGH conjugate, a buffer, sugar alcohol, and surfactant (Korean Application Publication No. 10-2015-0035681).

However, as production of liquid formulations of the long-acting hGH conjugates further comprising a preservative is still in demand, the present inventors attempted to develop an optimized liquid formulation of the long-acting hGH conjugate comprising a preservative as shown below. Specifically, effects of preservatives added for multiple administration on stability of the low-concentration hGH conjugate were examined.

The compositions shown in Table 1 below were used as liquid formulations of the long-acting hGH conjugates and stored at 4°C and 25°C for 0 to 3 months and analyzed using ion exchange chromatography (IE-HPLC) and size exclusion chromatography. IE-HPLC (%) and SE-HPLC (%) in Tables 2 and 3 are (Area %/Start Area %), indicating a residual rate of the long-acting hGH conjugate relative to an initial value. Table 2 shows IE-HPLC and SE-HPLC residual rates of the low-concentration long-acting hGH conjugates stored at 4°C, whereas Table 3 shows the same stored at 25°C.

**[Table 1]**

| | **Protein Conc.** | **Buffer Solution** | **pH** | **Sugar Alcohol** | **Surfactant** | **Preservative** |
|---|---|---|---|---|---|---|
| # 1 | 10.0 mg/mL | 20 mM sodium citrate | 5.2 | 4% mannitol | 0.005% polysorbate 80 | - |
| #2 | 10.0 mg/mL | 20 mM sodium citrate | 5.2 | 4% mannitol | 0.005% polysorbate 80 | 0.3% *m*-cresol |
| #3 | 10.0 mg/mL | 20 mM sodium citrate | 5.2 | 4% mannitol | 0.005% polysorbate 80 | 0.3% phenol |
| #4 | 10.0 mg/mL | 20 mM sodium citrate | 5.2 | 4% mannitol | 0.005% polysorbate 80 | 0.9% benzyl alcohol |

**[Table 2]**

| | **IE-HPLC (%)** | | | **SE-HPLC (%)** | | |
|---|---|---|---|---|---|---|
| | **Initial** | **1 month** | **3 months** | **Initial** | **1 month** | **3 months** |
| # 1 | 100.0 | 97.1 | 95.7 | 100.0 | 99.8 | 99.6 |
| #2 | 100.0 | 97.2 | 95.9 | 100.0 | 99.2 | 98.8 |
| #3 | 100.0 | 97.4 | 96.1 | 100.0 | 99.5 | 99.3 |
| #4 | 100.0 | 97.3 | 96.0 | 100.0 | 99.3 | 98.9 |

**[Table 3]**

| | **IE-HPLC (%)** | | | | **SE-HPLC (%)** | | | |
|---|---|---|---|---|---|---|---|---|
| | **Initial** | **2 weeks** | **1 month** | **3 months** | **Initial** | **2 weeks** | **1 month** | **3 months** |
| # 1 | 100.0 | 93.5 | 90.6 | 87.4 | 100.0 | 99.7 | 99.5 | 98.4 |
| #2 | 100.0 | 93.4 | 90.4 | 86.7 | 100.0 | 98.8 | 98.1 | 96.9 |
| #3 | 100.0 | 93.7 | 90.5 | 86.7 | 100.0 | 99.3 | 99.2 | 98.0 |
| #4 | 100.0 | 93.5 | 90.4 | 86.3 | 100.0 | 98.8 | 98.8 | 97.6 |

As shown in the results above, as a result of IE-HPLC and SE-HPLC after storage at 4°C, when the concentration of the long-acting hGH conjugate was 10.0 mg/mL, there was no significant difference between the stability of the conjugate which further comprises m-cresol, phenol, or benzyl alcohol (#2 to #4 of Table 2) as a preservative and that of the conjugate without any preservative (#1 of Table 2).

Additionally, as a result of IE-HPLC and SE-HPLC after storage at 25°C, when the concentration of the long-acting hGH conjugate was 10.0 mg/mL, the stability of the conjugate which further comprises a preservative (#2 to #4 of Table 3) slightly decreased, which is not a significant difference, compared to that of the conjugate without any preservative (#1 of Table 3).

When stored at 4°C and 25°C, the stabilities of low-concentration long-acting hGH conjugates according to a type of preservative were similar.

### Example 2: Evaluation of stability of liquid formulation of high-concentration long-acting hGH conjugate according to preservatives

Based on the liquid formulation confirmed in Example 1, effects of preservatives added for multiple administration on stability of a high-concentration long-acting hGH conjugate were examined.

The compositions shown in Table 4 below were used as liquid formulations of the long-acting hGH conjugates and stored at 4°C and 25°C for 0 to 3 months and analyzed using ion exchange chromatography (IE-HPLC) and size exclusion chromatography (SE-HPLC). IE-HPLC (%) and SE-HPLC (%) in Tables 5 and 6 are (Area %/Start Area %), indicating a residual rate of the long-acting hGH conjugate relative to an initial value. Table 5 shows IE-HPLC and SE-HPLC residual rates of the high-concentration long-acting hGH conjugates after storage at 4°C, whereas Table 6 shows the same after storage at 25°C.

**[Table 4]**

| | **Protein Conc.** | **Buffer Solution** | **pH** | **Sugar Alcohol** | **Surfactant** | **Preservative** |
|---|---|---|---|---|---|---|
| # 1 | 58.5 mg/mL | 20 mM sodium citrate | 5.2 | 4% mannitol | 0.005% polysorbate 80 | - |
| #2 | 58.5 mg/mL | 20 mM sodium citrate | 5.2 | 4% mannitol | 0.005% polysorbate 80 | 0.3% *m*-cresol |
| #3 | 58.5 mg/mL | 20 mM sodium citrate | 5.2 | 4% mannitol | 0.005% polysorbate 80 | 0.3% phenol |
| #4 | 58.5 mg/mL | 20 mM sodium citrate | 5.2 | 4% mannitol | 0.005% polysorbate 80 | 0.9% benzyl alcohol |

**[Table 5]**

| | **IE-HPLC (%)** | | | **SE-HPLC (%)** | | |
|---|---|---|---|---|---|---|
| | **Initial** | **1 month** | **3 months** | **Initial** | **1 month** | **3 months** |
| # 1 | 100.0 | 97.6 | 96.2 | 100.0 | 99.9 | 99.7 |
| #2 | 100.0 | 97.2 | 96.1 | 100.0 | 99.4 | 99.0 |
| #3 | 100.0 | 97.3 | 96.1 | 100.0 | 99.8 | 99.4 |
| #4 | 100.0 | 97.3 | 96.1 | 100.0 | 99.2 | 98.5 |

**[Table 6]**

| | **IE-HPLC (%)** | | | | **SE-HPLC (%)** | | | |
|---|---|---|---|---|---|---|---|---|
| | **Initial** | **2 weeks** | **1 month** | **3 months** | **Initial** | **2 weeks** | **1 month** | **3 months** |
| # 1 | 100.0 | 94.1 | 91.1 | 87.0 | 100.0 | 99.4 | 99.3 | 98.1 |
| #2 | 100.0 | 93.7 | 90.8 | 86.8 | 100.0 | 98.6 | 97.8 | 96.1 |
| #3 | 100.0 | 93.9 | 90.6 | 86.9 | 100.0 | 99.0 | 98.6 | 97.1 |
| #4 | 100.0 | 93.5 | 90.8 | 86.2 | 100.0 | 98.7 | 98.5 | 97.0 |

As shown in the results above, as a result of IE-HPLC and SE-HPLC analysis after storage at 4°C, when the concentration of the long-acting hGH conjugate was 58.5 mg/mL, there was no significant difference between the stability of the conjugate which comprises m-cresol, phenol, or benzyl alcohol (#2 to #4 of Table 5) as a preservative and that of the conjugate without any preservative (#1 of Table 5).

Additionally, as a result of IE-HPLC and SE-HPLC after storage at 25°C, when the concentration of the long-acting hGH conjugate was 58.5 mg/mL, the stability of the conjugate which comprises a preservative (#2 to #4 of Table 6) slightly decreased, which is not a significant difference, compared to that of the conjugate without any preservative (#1 of Table 6).

When stored at 4°C and 25°C, the stabilities of high-concentration long-acting hGH conjugates according to a type of preservative were similar.

In particular, as shown in the results of Examples 1 and 2, when the concentration of the long-acting hGH conjugate is between 10.0 mg/mL and 58.5 mg/mL under a condition of 20 mM sodium citrate at pH 5.2, 4% mannitol, and 0.005% polysorbate 80, the conjugate showed an excellent stability even though it comprised m-cresol, phenol, or benzyl alcohol as a preservative.

While the present invention has been described with reference to the particular illustrative embodiments, it will be understood by those skilled in the art to which the present invention pertains that the present invention may be embodied in other specific forms without departing from the technical spirit or essential characteristics of the present invention. Therefore, the embodiments described above are considered to be illustrative in all respects and not restrictive. Furthermore, the scope of the present invention is defined by the appended claims rather than the detailed description, and it should be understood that all modifications or variations derived from the meanings and scope of the present invention and equivalents thereof are included in the scope of the appended claims.

## Claims

1. A liquid formulation of a long-acting human growth hormone (hGH) conjugate, comprising:
(i) a long-acting hGH conjugate in which the hGH is linked to an immunoglobulin Fc region; and
(ii) an albumin-free stabilizer comprising a buffer solution, sugar alcohol, non-ionic surfactant, and preservative, without an isotonic agent.

2. The liquid formulation of claim 1, wherein the long-acting hGH conjugate is in the form of a fusion protein, in which hGH and an immunoglobulin Fc region are linked via a non-peptidyl polymer or a recombination technique.

3. The liquid formulation of claim 2, wherein the non-peptidyl polymer is selected from the group consisting of polypropylene glycol, polyethylene glycol, a copolymer of ethylene glycol and propylene glycol, polyoxyethylated polyol, polyvinyl alcohol, polysaccharide, dextran, polyvinyl ethyl ether, a biodegradable polymer such as polylactic acid (PLA) and polylactic-glycolic acid (PLGA), a lipid polymer, chitins, hyaluronic acid, and a combination thereof.

4. The liquid formulation of claim 2, wherein the non-peptidyl polymer is polyethylene glycol.

5. The liquid formulation of claim 1, wherein the buffer is a citrate buffer or acetate buffer.

6. The liquid formulation of claim 1, wherein the buffer has a pH in a range of 4.0 to 7.0.

7. The liquid formulation of claim 1, wherein the sugar alcohol is at least one selected from the group consisting of mannitol, sucrose, and sorbitol.

8. The liquid formulation of claim 1 or 7, wherein the sugar alcohol has a concentration in a range of 1% (w/v) to 10% (w/v) relative to the total volume of the liquid formulation.

9. The liquid formulation of claim 1, wherein the non-ionic surfactant is polysorbate or poloxamer.

10. The liquid formulation of claim 1 or 9, wherein the non-ionic surfactant has a concentration in a range of 0.001% (w/v) to 0.1% (w/v) relative to the total volume of the liquid formulation.

11. The liquid formulation of claim 1, wherein the preservative is at least one selected from the group consisting of m-cresol, phenol, and benzyl alcohol.

12. The liquid formulation of claim 1 or 11, wherein the preservative has a concentration in a range of 0.01% to 1.0% (w/v) relative to the total volume of the liquid formulation.

13. The liquid formulation of claim 1, wherein the liquid formulation is for multiple administration.

14. The liquid formulation of claim 1, wherein the long-acting hGH conjugate has a concentration in a range of 1 mg/mL to 100 mg/mL.

15. The liquid formulation of claim 1, wherein the stabilizer further comprises at least one selected from the group consisting of saccharides, polyhydric alcohol, and amino acids.

16. The liquid formulation of claim 1, wherein the hGH is hGH having the same amino acid sequence as that of native hGH, hGH having a modification on the amino acid sequence of the native hGH, or a derivative having a similar activity to that of the native hGH.

17. The liquid formulation of claim 1, wherein the liquid formulation of the long-acting hGH conjugate comprises:
(i) a long-acting hGH conjugate in which the hGH is linked to an immunoglobulin Fc region; and
(ii) an albumin-free stabilizer consisting of a buffer solution, sugar alcohol, non-ionic surfactant, and preservative.

18. The liquid formulation of claim 1 or 17, wherein the buffer is a citrate buffer, the sugar alcohol is mannitol, the surfactant is polysorbate 80, and the preservative is selected from the group consisting of *m*-cresol, phenol, and benzyl alcohol.

19. The liquid formulation of claim 1, wherein the immunoglobulin Fc region is derived from IgG, IgA, IgD, IgE, or IgM.

20. The liquid formulation of claim 1, wherein each domain of the immunoglobulin Fc region is a hybrid of domains with different origins derived from an immunoglobulin selected from the group consisting of IgG, IgA, IgD, IgE, and IgM.

21. The liquid formulation of claim 1, wherein the immunoglobulin Fc region is a dimer or multimer composed of single-chain immunoglobulins consisting of domains with the same origin.

22. The liquid formulation of claim 1, wherein the immunoglobulin Fc region is an aglycosylated human IgG4 Fc region.

23. The liquid formulation of claim 1, wherein the formulation is for treating pituitary dwarfism, growth hormone deficiency, Prader-Willi syndrome, or idiopathic short stature.

24. A method for preparing the formulation of claim 1, comprising mixing the long-acting hGH conjugate in which the hGH is linked to the immunoglobulin Fc region with a buffer, sugar alcohol, non-ionic surfactant, and preservative.
